# EUROPEAN PATENT APPLICATION

(11) **EP 3 290 040 A1**
(43) Date of publication of application: **07.03.2018**
(21) Application number: 16786504.7
(22) Date of filing: 27.04.2016
(51) Int. Cl.: A61K 31/551, A61P 27/02, A61P 43/00

(54) **PROPHYLACTIC AND THERAPEUTIC AGENT FOR COMPLICATIONS AFTER CATARACT SURGERY**

(30) Priority: 27.04.2015 JP 2015089918
(71) Applicant: Honjo, Megumi, Tokyo 115-0053 (JP); Kowa Company, Ltd., Nagoya-shi, Aichi 460-8625 (JP)
(72) Inventor: HONJO, Megumi, Tokyo 115-0053 (JP); OHTA, Masayuki, Higashimurayama-shi Tokyo 189-0022 (JP)
(74) Representative: Adam, Holger
(86) International application number: PCT/JP2016/063149
(87) International publication number: WO 2016/175228

(57) **Abstract**

Provided is a novel drug for preventing and treating complications after cataract surgery such as secondary cataract and anterior capsular contraction occurring after cataract surgery.

The prophylactic and therapeutic agent according to the present invention for complications after cataract surgery such as secondary cataract and anterior capsular contraction comprises, as an active ingredient, 4-fluoro-5-{[(2S)-2-methyl-1,4-diazepan-1-yl]sulfonyl}isoqu inoline (ripasudil), a salt thereof or a solvate of the same.

## Description

### Technical Field

The present invention relates to a novel medicament useful for the prevention and treatment of complications after cataract surgery, such as secondary cataract and anterior capsule contraction.

### Background Art

The crystalline lens refracts light entering into the eye to form an image on the retina and is responsible for the image formation system with the cornea. The anterior surface touches the posterior iris surface through the aqueous humor in the posterior chamber and the posterior surface touches the vitreous body. The crystalline lens is a transparent avascular tissue, and surrounded with a transparent thin membrane called the lens capsule. The anterior surface of the lens capsule is called the anterior capsule, the posterior surface is called the posterior capsule, and the part of the disc edge is called the equator. The zonule of Zinn connects the equator, and is involved in regulating the thickness of the crystalline lens.

The lens epithelial cells line the inside of the lens capsule, and cell division is observed near the equator (epithelial cells in the proliferative zone). The epithelial cells in the equator take the form of an elongated rectangle to differentiate into fiber cells, which are extended to the posterior capsule, transferred towards the central part of the crystalline lens, compressed to the central part of the crystalline lens and dehydrated to form the nucleus of the crystalline lens. As described above, the posterior capsule is fibers, and different from the anterior capsule in this respect. In general, the lens epithelial cells are moderately proliferated, and it is believed that the crystalline lens become heavier and thicker with increasing age.

In addition, spinelike processes, ridge processes and gap junctions exist between fiber cells into which epithelial cells are differentiated, and when there are abnormalities in epithelial cell division and abnormalities in extension to the posterior capsule, opacification due to lens fibers can occur.

Cataracts are caused by lens opacity, and, because incident light is scattered by lens opacity, symptoms such as blurred vision, double vision and intolerance of light emerge, and visual acuity is reduced with progression and cannot be corrected even with eyeglasses. The causes of cataract include congenital conditions, trauma, atopy, drugs, radiation and the like; however, most of the cataracts are caused by aging, which are generally called senile cataract (age-related cataract) and are believed to be mainly caused by the progression of cortical opacity (cortical cataract) and nuclear sclerosis (nuclear cataract). The surface of light which passes through the crystalline lens varies depending on the size of the pupil, and thus when opacification occurs in the site through which light does not pass, there are almost no subjective symptoms. When observing the crystalline lens in a pupil dilation test (mydriasis test), however, cataracts are detected in people in their forties in the earlier cases, and in the majority of people in their eighties.

In the very early stage of cataract, the progression can be delayed with an ophthalmic preparation in some cases; however, the crystalline lens in which opacification has occurred once cannot be recovered, and for advanced cataract, a method in which the nucleus and cortex of the opaque crystalline lens are removed by a surgical operation (extracapsular lens extraction) and an intraocular lens is inserted is generally carried out. An example thereof is a method in which the anterior surface of the lens capsule (anterior capsule) is incised in a circular manner, the contents in the capsule are fragmented with ultrasonic and sucked, and an intraocular lens is inserted into the remaining capsule, and inserting an intraocular lens becomes easy by leaving the lens capsule.

This operation hardly causes pain, ends in a relatively short time, and restores visual acuity by inserting an intraocular lens, and thus has been used as a method for treating cataract. It is also said that the disease rate of senile cataract is 60 to 70% in people in their sixties and further almost 100% in people in their eighties or older. In the present situation in which aging society progresses, the importance of the treatment of cataract has increasingly risen, and operation methods and intraocular lenses have been improved.

After a period of time after the operation, however, the crystalline lens can become turbid again by, for example, the proliferation and migration to the posterior capsule of the lens cells remaining in the capsule. When the opacification progresses, visual function declines and cataract symptoms emerge in some cases. This is called "secondary cataract", and the incidence rate is reported to be above 19%. However, a complete method for preventing and treating secondary cataract has not been established until now.

As the onset mechanism of secondary cataract, it is mainly believed that the lens epithelial cells which have not been completely removed by extracapsular lens extraction proliferate and migrate to the posterior capsule, thereby giving rise to posterior capsule opacification (PCO), or Soemmerring's ring cataract, which has a ring-shaped opacity, and Elschnig's pearls, which have a pearl-like opacity, are formed by, for example, the abnormal proliferation of lens epithelial cells remaining in the equator. It is impossible to completely remove lens epithelial cells in a cataract surgery and it is difficult to completely prevent secondary cataract. It is demanded that a medicine for the prevention and treatment of secondary cataract be developed.

In addition, the window of the lens capsule obtained by incising the anterior surface of the lens capsule (anterior capsule) in a circular manner in the cataract surgery described above becomes small after the operation in some cases. Such symptom is called "anterior capsule contraction". This occurs because the lens cells remaining around the incised window are changed to fibrous cells by for example an inflammatory reaction and proliferated to narrow the incised circular window as a purse. This does not normally affect visual function in most cases; however, when the size of the window is increased to cover the central part of the pupil, light is difficult to enter into the eyes, which causes a declined visual function. The "anterior capsule contraction" is also a disease occurring after a cataract surgery, and an effective method for the prevention and treatment thereof has not been developed as is the case with secondary cataract, and it is demanded that a medicine for the prevention and treatment of anterior capsule contraction be developed.

Various substances have been reported as secondary cataract inhibitors. There have been reported the agents for inhibiting secondary cataract which are based on an anti-proliferative action on lens epithelial cells; for example, N-(3,4-dimethoxycinnamoyl)anthranilic acid (generic name: Tranilast), which is known as a therapeutic agent for allergic diseases such as bronchial asthma and allergic rhinitis, inhibits the proliferation of lens epithelial cells (see Patent Document 1); transforming growth factor-β (TGF-β) restricts excessive proliferation of lens epithelial cells (see Patent Document 2); serine protease enzyme precursors such as Lys-plasminogen promotes the separation of lens epithelial cells from the lens capsule and prevents the proliferation of lens epithelial cells (see Patent Document 3); and cell death receptor ligands such as Fas ligand induce apoptosis of lens epithelial cells (see Patent Document 4).

In addition, there have been reported agents for inhibiting secondary cataract which are based on an inhibitory action on migration or extension of lens epithelial cells; for example, ethylenediaminetetraacetic acid prevents the migration of lens epithelial cells and inhibits their adhesion to the posterior capsule (see Patent Document 5); non-peptidic cell-adhesion inhibitors such as 2-piperazinone derivatives inhibit the extension toward and superposition of lens epithelial cells onto the posterior capsule (see Patent Document 6); a polypeptide having cell-adhesion inhibiting activity and a lactic acid-glycolic acid polymer inhibit the extension toward and superposition of lens epithelial cells onto the posterior capsule (see Patent Document 7); peptides such as Arg-Gly-Asp have cell-adhesion inhibiting activity and inhibit the extension toward and superposition of lens epithelial cells onto the posterior capsule (see Patent Document 8).

Furthermore, it has also been reported that H-7, a serine threonine kinase inhibitor, was effective in resolving Soemmerring's rings, but did not have any effect on posterior capsule opacification (PCO) (see Non-Patent Document 1).

In the meantime, it has been reported that Rho-kinase (Rho-Associated, Coiled-Coil Containing Protein Kinase: ROCK) inhibitors (also referred to hereinafter as ROCK inhibitors), such as fasudil and Y-27632, are used for the treatment of glaucoma and others (see Patent Document 9, for example), and that they are useful as an agent for the prevention or treatment of axial myopia (see Patent Document 10).

It has also been reported that ROCK inhibitors HF and Y-27632 inhibited the contraction induced by cytokines such as PDGF-BB and TGF-β2 in bovine lens cells (see Non-Patent Document 2); that ROCK inhibitors Y-27632, HA-1077, H-1152, and ML-7 inhibit TGF-β induced transdifferentiation and contraction of myofibroblasts, and are useful against postoperative scarring of glaucoma filtration surgery (see Non-Patent Document 3); that a ROCK inhibitor Y-27632 does not promote proliferation, but promotes adhesiveness and is useful as an anti-scarring agent after glaucoma filtration surgery (see Non-Patent Document 4); and that a ROCK inhibitor fasudil inhibits the shrinkage of collagen gels containing vitreous cells and is useful for the treatment of vitreoretinal diseases (see Non-Patent Document 5).

It has not been reported, however, that ROCK inhibitors are useful for the prevention and treatment of complications after cataract surgery, such as secondary cataract and anterior capsule contraction.

### Citation List

### Patent Document

Patent Document 1: WO 98/16214 A
Patent Document 2: JP 8-502033 A
Patent Document 3: JP 2002-502821 A
Patent Document 4: JP 2004-518649 A
Patent Document 5: JP 8-175984 A
Patent Document 6: JP 9-235239 A
Patent Document 7: JP 9-291040 A
Patent Document 8: JP 10-17487 A
Patent Document 9: WO 00/09162 A
Patent Document 10: WO 2010/010702 A

### Non-Patent Document

Non-Patent Document 1: Baohe Tian, et al., J. Ocular Pharma. Ther., 26(6), 2010, 533-539.
Non-Patent Document 2: Kumiko Hirayama, et al., IOVS, 45(11), 2004, 3896-3903.
Non-Patent Document 3: Tobias Meyer-ter-Vehn, et al., IOVS, 47(11), 2006, 4895-4904.
Non-Patent Document 4: Megumi Honjo, et al., IOVS, 48 (12), 2007, 5549-5557.
Non-Patent Document 5: Takeshi Kita, et al., PNAS, 105(45), 2008, 17504-17509.

### Summary of the Invention

### Problems to be Solved by the Invention

The present invention provides a novel medicament for the prevention and treatment of complications after cataract surgery, such as secondary cataract and anterior capsule contraction, which develop after cataract surgery, particularly after extracapsular lens extraction for surgical removal of the opacified lens.

The present invention also provides a novel medicine as a cytostatic agent, particularly as a cytostatic agent for lens epithelial cells after a cataract surgical operation.

The present invention further provides a novel medicine as a collagen gel contraction inhibitor, particularly a collagen gel contraction inhibitor for use after a cataract surgical operation.

### Means for Solving the Problems

The present inventors have investigated various actions of 4-fluoro-5-{[(2S)-2-methyl-1,4-diazepan-1-yl]sulfonyl}isoqu inoline (also referred to hereinafter as Ripasudil) having the following structure:

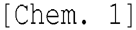

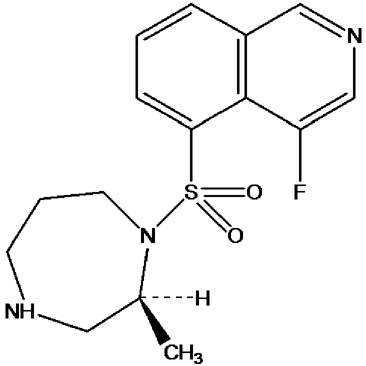

The therapeutic drug for the treatment of glaucoma and ocular hypertension that contains ripasudil hydrochloride dihydrate as an active ingredient has already been approved under the trade name "Glanatec"® for manufacturing and marketing.

The present inventors have made an investigation on actions of Ripasudil on the inhibition of proliferation of human lens epithelial cells and of fibroblasts, and on collagen gel contraction. From the results of this investigation, Ripasudil was found to have actions of inhibiting the cell proliferation of these cell types and of inhibiting the collagen gel contraction, and was ascertained to be useful as a medicament for the prevention and treatment of complications after cataract surgery, such as secondary cataract and anterior capsule contraction.

Since, as is apparent from these findings, Ripasudil exerts not only an action of inhibiting the proliferation of the lens epithelial cells that have remained by their incomplete removal when the nucleus and cortex of a lens are extracted after the anterior capsule resection in a surgical procedure for cataract, but also an action of inhibiting the shrinkage of collagen gel, it has turned out that Ripasudil is effective in the prevention and/or treatment of complications after cataract surgery, such as secondary cataract and anterior capsule contraction.

Accordingly, the present invention relates to an agent for the prevention and treatment of complications after cataract surgery, such as secondary cataract and anterior capsule contraction containing 4-fluoro-5-{[(2S)-2-methyl-1,4-diazepan-1-yl]sulfonyl}isoqu inoline (Ripasudil) or a salt thereof, or a solvate thereof, or a solvate of the salt thereof as an active ingredient.

The present invention also relates to a cytostatic agent, containing Ripasudil or a salt thereof, or a solvate thereof, or a solvate of the salt thereof as an active ingredient, preferably a cytostatic agent for lens epithelial cells, and more preferably a cytostatic agent for lens epithelial cells after a cataract surgical operation.

The present invention also relates to a collagen gel contraction inhibitor, containing Ripasudil or a salt thereof, or a solvate thereof, or a solvate of the salt thereof as an active ingredient, and preferably a collagen gel contraction inhibitor for use after a cataract surgical operation.

### Effects of the Invention

According to the present invention, there can be provided a novel agent for the prevention and/or treatment of complications after cataract surgery, such as secondary cataract and anterior capsule contraction. Ripasudil or a salt thereof, or a solvate thereof, or a solvate of the salt thereof used in the present invention, can be used as a cytostatic agent, preferably a cytostatic agent for lens epithelial cells, more preferably a cytostatic agent for lens epithelial cells after a cataract surgical operation, and also as a collagen gel contraction inhibitor, preferably a collagen gel contraction inhibitor for use after a cataract surgical operation.

The complications after cataract surgery such as secondary cataract and anterior capsule contraction occurring after a cataract surgery disrupt the recovery of visual function after a surgical operation, and are serious diseases for patients. No drug effective in preventing or treating the diseases has, however, developed until now, and there has been only treatment by a laser. The present invention provides a novel drug, which is able to prevent and treat the diseases by an easy administration treatment such as ocular instillation.

Furthermore, there is apprehension about an increasing number of patients with senile cataract due to an aging society, however, the drug of the present invention is able to simply and effectively inhibit complications after cataract surgery such as secondary cataract and anterior capsule contraction occurring after a cataract surgery, and the patients are able to have a cataract surgery without anxiety.

### Brief Description of Drawings

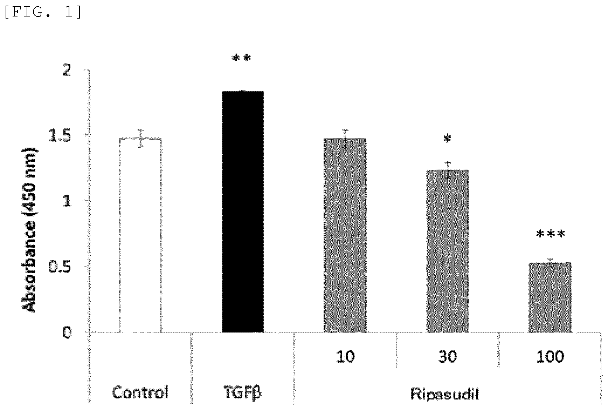

FIG. 1 shows results of the investigation of actions of Ripasudil on cell proliferation (DNA synthesis ability) of human lens epithelial cells (SRA 01/04). The results are expressed as mean ± standard deviation, with * indicating a significant difference at p < 0.05, ** indicating a significant difference at p < 0.01, and *** indicating a significant difference at p < 0.001.

### Modes for Carrying Out the Invention

In the following, aspects of the present invention will be described in more detail.

Accordingly, the present invention encompasses the inventions described below:
(1) An agent for prevention and/or treatment of complications after cataract surgery, such as secondary cataract and anterior capsule contraction, containing 4-fluoro-5-{[(2S)-2-methyl-1,4-diazepan-1-yl]sulfonyl}isoqu inoline, or a salt thereof, or a solvate thereof, or a solvate of the salt thereof as an active ingredient.
(2) The agent for prevention and/or treatment according to (1) above, wherein the solvate is a hydrate.
(3) The agent for prevention and/or treatment according to (1) or (2) above, wherein the treatment is to block the progression of opacification or contraction of the lens capsule.
(4) The agent for prevention and/or treatment according to any one of (1) to (3) above, wherein the agent for prevention and/or treatment is administered from during a cataract surgery or after a cataract surgery.
(5) The agent for prevention and/or treatment according to (4) above, wherein the cataract surgery includes extracapsular lens extraction.
(6) A pharmaceutical composition for prevention and/or treatment of complications after cataract surgery, such as secondary cataract and anterior capsule contraction, containing Ripasudil or a salt thereof, or a solvate thereof, or a solvate of the salt thereof, and a pharmaceutically acceptable carrier.
(7) The pharmaceutical composition according to (6) above, wherein the solvate is a hydrate.
(8) The pharmaceutical composition according to (6) or (7) above, wherein the treatment is to block the progression of opacification or contraction of the lens capsule.
(9) The pharmaceutical composition according to any one of (6) to (8) above, wherein the pharmaceutical composition for prevention and/or treatment is administered from during a cataract surgery or after a cataract surgery.
(10) The pharmaceutical composition according to (9) above, wherein the cataract surgery includes extracapsular lens extraction.
(11) An ophthalmic preparation for prevention and/or treatment of complications after cataract surgery, such as secondary cataract and anterior capsule contraction, containing Ripasudil or a salt thereof, or a solvate thereof, or a solvate of the salt thereof, and an acceptable carrier for ophthalmic preparation.
(12) The ophthalmic preparation according to (11) above, wherein the solvate is a hydrate.
(13) The ophthalmic preparation according to (11) or (12) above, wherein the treatment is to block the progression of opacification or contraction of the lens capsule.
(14) The ophthalmic preparation according to any one of (11) to (13) above, wherein the ophthalmic preparation for prevention and/or treatment is administered from during a cataract surgery or after a cataract surgery.
(15) The ophthalmic preparation according to (14) above, wherein the cataract surgery includes extracapsular lens extraction.
(16) A cytostatic agent, containing 4-fluoro-5-{[(2S)-2-methyl-1,4-diazepan-1-yl]sulfonyl}isoqu inoline or a salt thereof, or a solvate thereof, or a solvate of the salt thereof as an active ingredient.
(17) The cytostatic agent according to (16) above, wherein the solvate is a hydrate.
(18) The cytostatic agent according to (16) or (17) above, wherein the cytostatic agent is a cytostatic agent for lens epithelial cells.
(19) The cytostatic agent according to any one of (16) to (18) above, wherein the cytostatic agent is a cytostatic agent for lens epithelial cells after a cataract surgical operation.
(20) The cytostatic agent according to any one of (16) to (19) above, wherein the cytostatic agent blocks the opacification of the lens after a cataract surgical operation.
(21) The cytostatic agent according to any one of (16) to (20) above, wherein the cytostatic agent inhibits the morphogenesis of the crystalline lens after a cataract surgical operation.
(22) The cytostatic agent according to any one of (16) to (21) above, wherein the cytostatic agent is for prevention and/or treatment of complications after cataract surgery, such as secondary cataract and anterior capsule contraction.
(23) A collagen gel contraction inhibitor, containing 4-fluoro-5-{[(2S)-2-methyl-1,4-diazepan-1-yl]sulfonyl}isoqu inoline or a salt thereof, or a solvate thereof, or a solvate of the salt thereof as an active ingredient.
(24) The collagen gel contraction inhibitor according to (23) above, wherein the solvate is a hydrate.
(25) The collagen gel contraction inhibitor according to (23) or (24) above, wherein the collagen gel contraction inhibitor is a collagen gel contraction inhibitor for use after a cataract surgical operation.
(26) The collagen gel contraction inhibitor according to any one of (23) to (25) above, wherein the collagen gel contraction inhibitor blocks the collagen gel contraction after a cataract surgical operation.
(27) The collagen gel contraction inhibitor according to any one of (23) to (26) above, wherein the collagen gel contraction inhibitor inhibits the morphogenesis of the crystalline lens after a cataract surgical operation.
(28) The collagen gel contraction inhibitor according to any one of (23) to (27) above, wherein the collagen gel contraction inhibitor is for prevention and/or treatment of complications after cataract surgery, such as secondary cataract and anterior capsule contraction.

It is known that secondary cataract develops due to proliferation, differentiation into fiber cells, extension and migration of lens epithelial cell after a cataract surgical operation. That is, it is known that the morphogenesis of the crystalline lens by lens epithelial cells after a cataract surgical operation causes opacity. In particular, the proliferation of the lens epithelial cells that have remained after a cataract surgical operation is considered to be a major cause of secondary cataract. Therefore, Ripasudil used in the present invention is useful in the prevention and/or treatment of secondary cataract because Ripasudil is found to have a cytostatic action, including cytostatic action on lens epithelial cells.

In addition, it is known that anterior capsule contraction develops due to the contraction of collagen in the lens capsule, and Ripasudil used in the present invention is useful in the prevention and/or treatment of anterior capsule contraction after a cataract surgical operation because Ripasudil is found to exert an action of inhibiting collagen gel contraction.

Ripasudil, which is employed in the present invention, is a cerebrovascular treatment agent and is a compound having substance P antagonism, leukotriene D4 antagonism, and ROCK inhibiting action, and can be produced by known methods, for example, a method described in WO 99/20620.

Salts of Ripasudil include, for example, salts of inorganic acids such as hydrochloric acid, sulfuric acid, nitric acid, hydrofluoric acid, and hydrobromic acid, or salts with organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid. Among these salts, hydrochloride is especially preferred.

Ripasudil or its salt may exist not only as an unsolvated form, but also as a hydrate or a solvate. Although hydrates are preferable, all crystal forms and hydrates or solvates of Ripasudil or its salt are encompassed by the present invention.

Ripasudil or a salt thereof, or a solvate thereof, or a solvate of the salt thereof has cytostatic action, inhibitory action on collagen contraction and the like, and as shown in the Examples section which follows, and is useful as an agent for the prevention and/or treatment of complications after cataract surgery, such as posterior capsule opacification, Soemmerring's ring cataract, Elschnig's pearl formation, secondary cataract, and anterior capsule contraction, and decline in visual acuity and visual function due to these complications.

In the present invention, "after cataract surgery" means after the treatment of cataract such as extracapsular lens extraction and after a surgical operation to improve visual function due to cataract. In the present invention, "after cataract surgery" is also referred to as "after a cataract operation" or "after a cataract surgical operation".

In the present invention, "complications after cataract surgery" include diseases accompanied by various symptoms which develop or can develop after the treatment of cataract such as extracapsular lens extraction and a surgical operation to improve visual function due to cataract, more specifically, diseases which develop or can develop due to, for example, the proliferation, differentiation, extension and migration of the lens fiber cells derived from lens epithelial cells which have not been completely removed by the above surgical operation. Typical examples of complications after cataract surgery include secondary cataract, anterior capsule contraction and the like.

In the present invention, "prevention/treatment" means prevention and/or treatment. The treatment herein includes the inhibition of the progression of opacification or contraction of the lens capsule. In addition, the prevention is not necessarily supposed to develop the diseases, and also includes inhibiting the possibility of developing the diseases. The prevention in the present invention further includes, when the nucleus and cortex of the crystalline lens are extracted after excising the anterior capsule in a cataract surgery, immediate administration after excising the anterior capsule in order to inhibit proliferation, differentiation, extension and migration of the remaining lens epithelial cells which have not been completely removed.

When Ripasudil or a salt thereof, or a solvate thereof, or a solvate of the salt thereof is formulated into pharmaceutical preparations, these preparations can be prepared according to known methods. For example, pharmaceutical preparations of Ripasudil or a salt thereof, or a solvate thereof, or a solvate of the salt thereof can be prepared with reference to examples of the pharmaceutical preparations described, for example, in international patent publications (such as WO 00/09162 and WO 97/23222).

When pharmaceutical preparations of Ripasudil or a salt thereof, or a solvate thereof, or a solvate of the salt thereof are prepared, these preparations can be prepared according to known methods. For example, an ophthalmic preparation can be prepared using a tonicity agent, a buffer, a surfactant, a preservative, and others, as necessary. The pH of the ophthalmic preparation may be within a range that is acceptable to the ophthalmic preparation, and preferably is in the range of pH 4 to 8.

A preparation of the present invention is preferably used as an ophthalmic preparation, which may be any of an aqueous ophthalmic preparation, a non-aqueous ophthalmic preparation, an ophthalmic suspension, an ophthalmic emulsion, an eye ointment, and others. Such an ophthalmic preparation can be prepared as a composition suitable for the dosage form by mixing optionally a pharmaceutically acceptable carrier, for example, in the case of an ophthalmic preparation, a carrier acceptable to the ophthalmic preparation, such as a tonicity agent, a chelating agent, a stabilizer, a pH adjuster, a preservative, an antioxidant, a solubilizing agent, and a thickening agent, by (formulation) methods known to those skilled in the art.

The active ingredient used in the present invention is effective also in cases of eye drop administration, and can be used as an ophthalmic preparation. An ophthalmic preparation of the present invention contains the active ingredient used in the present invention, i.e., Ripasudil or a salt thereof, or a solvate thereof, or a solvate of the salt thereof, and an acceptable carrier for ophthalmic preparation.

When an ophthalmic preparation is prepared, the ophthalmic preparation can be prepared, for example, by dissolving or suspending any of the other desired above-described ingredients in an aqueous solvent such as sterile purified water or physiological saline or a non-aqueous solvent, for example, vegetable oils, such as cottonseed oil, soybean oil, sesame oil, and peanut oil, followed by adjustment to a predetermined osmotic pressure and then sterilization such as filter sterilization. When an ophthalmic ointment is prepared, it may include an ointment base in addition to the ingredients described above. The ointment base preferably includes, for example, oily bases such as petrolatum, liquid paraffin, and polyethylene; emulsion-type bases in which an oil phase and an aqueous phase are emulsified with a surfactant or the like; and water-soluble bases made of hydroxypropylmethyl cellulose, carboxymethyl cellulose, polyethylene glycol or the like, without being particularly limited thereto.

When Ripasudil or a salt thereof, or a solvate thereof, or a solvate of the salt thereof is used for the prevention and/or treatment of complications after cataract surgery, such as secondary cataract and anterior capsule contraction, the dosage amount varies depending on the body weight, age, gender, and symptoms of the patient, administration form, and frequency of administration, and the like. In usual cases, dosages amount for an adult are in the range of 0.025 to 10000 µg per day, preferably 0. 025 to 2000 µg per day, more preferably 0.1 to 2000 µg per day, and furthermore, 0.025 to 200 µg per day, in terms of (S)-(-)-1-(4-fluoro-5-isoquinolinesulfonyl)-2-methyl-1,4-ho mopiperazine or a salt thereof, or a solvate thereof, or a solvate of the salt thereof.

There is no particular limitation for the frequency of administration, and administration is preferably performed in one or several times, and in cases of a liquid ophthalmic preparation, one or several drops can be administered to the eye at a time.

In the following, the present invention is described more specifically with reference to Examples, but is not limited thereto in any way.

### Example 1

Human normal fibroblasts (Sanko Junyaku Co., Ltd.) were seeded at 2000 cells/well and cultured overnight. Next day, the medium (FGM-2 (Sanko Junyaku Co., Ltd.)) was exchanged (150 µL) and then Ripasudil was added in a volume of 10 µL to make final concentrations of 0.1 µM, 1.0 µM, and 10.0 µM. After culturing for 2 days, to the culture was added 10 µL of a BrdU solution (a 58.8-fold diluted solution, of which the stock solution had a 1000 times higher concentration), and the mixture was cultured for 2 hours. Fixation and antibody addition, washing and color development, and reaction stopping were performed according to the protocol of the Cell Proliferation ELISA BrdU kit (Roche). Then, a plate reader was used to measure OD540-620 to evaluate the action of Ripasudil on fibroblast proliferation. Table 1 shows percentage of BrdU incorporation under these conditions when the value of incorporation in the case of no added Ripasudil was set to be 100%.

**[Table 1]**

| ITEM | Concentration of Ripasudil (µM) | BrdU incorporation (% of control) |
|---|---|---|
| Cell proliferation | 0.1 | 80.0 |
| (Human normal fibroblasts) | 1.0 | 59.3 |
| | 10.0 | 10.9 |

The percent BrdU incorporation was 80.0% at 0.1 µM Ripasudil, 59.3% at 1.0 µM, and 10.9% at 10.0 µM, and from these results, it was found that Ripasudil has a significant cytostatic action.

### Example 2

Human lens epithelial cells (SRA 01/04 (RIKEN Cell Bank)) were cultured using 20% FBS-DMEM medium under conditions at 37°C and 5% CO₂.

The concentration of SRA 01/04 cells was adjusted using 2% FBS-DMEM medium, and the cells were seeded in a 96-well plate at 100 µL/well (5000 cells/well) and incubated for 24 hours under conditions at 37°C and 5% CO₂. The medium was exchanged to 5% FBS-DMEM medium, to which TGFβ was added at 10 ng/mL (final concentration) or Ripasudil at 10, 30, or 100 µM (final concentration), and then the cells were cultured for another 24 hours. The cell proliferation ELISA, BrdU (colorimetric) kit (Roche) was used to measure the amount of intracellular 5-bromo-2'-deoxyuridine (BrdU) incorporation at a measurement wavelength of 450 nm with Multiskan MS version 8.0 (LABSYSTEMS), thereby to evaluate the effect on the cell proliferation of SRA 01/04 cells.

The results are shown in FIG. 1. Ripasudil was observed to exhibit a dose-dependent inhibitory action on the DNA synthesis of human lens epithelial cells, in which a statistically significant decrease was shown at 30 µM.

This result, in combination with the results from Example 1 above, indicates that Ripasudil used in the present invention has an inhibitory action on proliferation of cells, including human lens epithelial cells.

### Example 3

### Inhibitory action on collagen gel contraction

HT-1080 cell suspension adjusted to a density of 150,000 to 200,000 cells/mL with a medium (medium: E'MEM (Sigma)/10% FBS/NEAA/PCSM), and a solution, obtained by mixing A, B and C solutions (A solution: Cellmatrix (Collagen Type I 3 mg/mL), B solution: concentrated culture medium, 10 x MEM Hank's culture medium, and C solution: buffer for reconstitution) of collagen gel culture kit (Nitta Gelatin Inc.) mixed at a ratio of 8 : 1 : 1 and cooling the obtained mixture, were mixed at a ratio of 1.4 : 1, and the obtained mixture was seeded in a 48 Well Plate at 500 µL/well and gelatinized in a CO₂ incubator to produce a cell-containing collagen gel. After gelation, 1 mL of the medium in which Ripasudil was adjusted to 0.1 µM, 1.0 µM or 10.0 µM was added on the gel, and an injection needle was inserted between the gel and the wall surface to release the gel from the wall surface, and the gel was incubated to stand in a CO₂ incubator. After two or three days, the contraction of the gel was observed, and photos were taken to evaluate the actions of Ripasudil.

When a contraction level was almost equal to that of a group to which Ripasudil was not added, (+) was used, (++) was used in the case where contraction was inhibited, and (+++) was used in the case where contraction was significantly inhibited.

The results are shown in Table 2 below.

**[Table 2]**

| ITEM | Ripasudil concentration (µM) | Inhibition of contraction |
|---|---|---|
| Collagen contraction ( HT1080 ) | 0.1 | + |
| | 1.0 | ++ |
| | 10.0 | +++ |

The action of Ripasudil on the inhibition of collagen gel contraction was assessed as (+) at 0.1 µM, (++) at 1.0 µM, and (+++) at 10.0 µM, and from these results, it was found that Ripasudil has a dose-dependent inhibitory action on collagen gel contraction.

According to the above, Ripasudil used in the present invention has the cytostatic action human lens epithelial cells, and can effectively inhibit the proliferation of the lens epithelial cells that have remained during cataract surgery, particularly during a surgical operation for the removal of the opacified lens, such as extracapsular lens extraction, and thus can be used as an agent for the prevention and/or treatment of complications after cataract surgery, such as secondary cataract, which are caused due to the proliferation of such lens epithelial cells.

In addition, Ripasudil used in the present invention has the inhibitory action on collagen gel contraction, and can be used as an agent for the prevention and/or treatment of complications after cataract surgery, such as anterior capsule contraction, which are caused due to the contraction of the lens capsule that has been incised during cataract surgery, particularly during a surgical operation for the removal of the opacified lens, such as extracapsular lens extraction.

### Industrial Applicability

Ripasudil or a salt thereof, or a solvate thereof, or a solvate of the salt thereof used in the present invention is useful as a medicament, such as an agent for the prevention and/or treatment of complications after cataract surgery, such as secondary cataract and anterior capsule contraction, a cytostatic agent, and a collagen gel contraction inhibitor, and thus has industrial applicability.

## Claims

1. An agent for prevention and/or treatment of complications after cataract surgery,
containing4-fluoro-5-{[(2S)-2-methyl-1,4-diazepan-1-yl]sulf onyl}isoquinoline or a salt thereof, or a solvate thereof, or a solvate of the salt thereof, as an active ingredient.

2. A cytostatic agent,
containing4-fluoro-5-{[(2S)-2-methyl-1,4-diazepan-1-yl]sulf onyl}isoquinoline or a salt thereof, or a solvate thereof, or a solvate of the salt thereof, as an active ingredient.

3. The cytostatic agent according to claim 2, wherein the cytostatic agent is for lens epithelial cells after a cataract surgical operation.

4. An inhibitor agent for collagen gel contraction, containing4-fluoro-5-{[(2S)-2-methyl-1,4-diazepan-1-yl]sulf onyl}isoquinoline or a salt thereof, or a solvate thereof, or a solvate of the salt thereof, as an active ingredient.

5. The inhibitor agent according to claim 4, wherein the inhibitor agent is for inhibiting the collagen gel contraction in lens epithelial cells after a cataract surgical operation.
